Europäisches Patentamt

**European Patent Office**

**Office européen des brevets**

(11) Publication number: **0 333 147**
**A1**

# EUROPEAN PATENT APPLICATION

(21) Application number: **89104549.4**

(22) Date of filing: **15.03.89**

(51) Int. Cl.⁴: **B65F 1/06** , **B65D 5/10** , **B65D 5/46**

(30) Priority: **18.03.88 IT 1811188**
**17.11.88 IT 1813288**
**10.03.89 IT 1810889**

(43) Date of publication of application:
**20.09.89 Bulletin 89/38**

(84) Designated Contracting States:
**AT BE CH DE ES FR GR IT LI NL**

(71) Applicant: **ALLPACK S.R.L.**
**Strada Pioppelle 3A**
**I-46029 Suzzara Mantova(IT)**

(72) Inventor: **Guaita, Giovanni**
**Via Pioppelle 5**
**I-46029 Suzzara Mantova(IT)**

(74) Representative: **Modiano, Guido et al**
**MODIANO, JOSIF, PISANTY & STAUB**
**Modiano & Associati Via Meravigli, 16**
**I-20123 Milan(IT)**

(54) **Disposable container.**

(57) The invention relates to a disposable container comprising an outer box-like body made of cardboard or the like with a lateral wall adapted to support a bag (11) rigidly associated therewith, a bottom and a closure device which is arranged at the face to be directed upwards, characterized in that the closure device comprises a first lid (23) which is adapted to associate with the face of the container to be directed upwards and has an opening (23C) adapted to allow the protrusion of the bag's inlet, and furthermore comprises a second lid (24) which is intended to superimpose on the first one and has a portion (25) adapted to be selectively locked in a position in which the opening provided in the first lid is closed or not closed.

Fig. 4

## DISPOSABLE CONTAINER

The present invention relates to a disposable container.

It is known that waste is progressively created in many workplaces and must be eliminated; this is the case, for example, of toxic hospital waste, constituted by pieces of gauze, syringes and the like, which the medical personnel discards into a container which is provided at the workplace and is subsequently removed by specifically assigned personnel when it is full.

Known containers comprise a plastic bag associated with an outer box-like body made of cardboard which has handles and comprises a lateral wall, a bottom and a closure device which is arranged at its upwardly directed base; when the bag is full it is closed by means of a lace and the container is then handled by gripping it by the handles of said outer box-like body.

A disadvantageous characteristic of known containers resides in the fact that during its use the closure device does not offer the inlet of the bag in the arrangement which is most appreciated by the users, and in that it furthermore lacks the necessary strength and, to an even greater extent, the characteristics of simplicity which would make it quick and easy to handle.

Known containers furthermore do not ensure that the bag will not separate from the outer box-like body, especially if the bottom of said box-like body deteriorates, and this fact makes them fully unsuitable for the treatment of toxic waste.

The aim of the present invention is therefore to provide a disposable container which has a closure device which can be adapted to any kind of wall of the outer box-like body and has considerable functional characteristics both during the step of filling the bag and during the steps of forming and of final closure.

An object of the present invention is to provide a container which gives the greatest assurance of solidity during handling.

This aim and this object are achieved by a disposable container according to the invention, comprising an outer box-like body made of cardboard or the like with a lateral wall adapted to support a bag rigidly associated therewith, a bottom and a closure device at the face intended to be directed upwards, characterized in that said closure device comprises a first lid which is adapted to associate with the face of the container to be directed upward, has an opening adapted to allow the bag's inlet to protrude, comprises a second lid intended to superimpose on the first one and has a portion adapted to be selectively locked in positions which close or do not close the opening provided in the first lid.

Further characteristics and advantages will become apparent from the description of two preferred but not exclusive embodiments of the invention, illustrated only by way of non-limitative example in the accompanying drawings, wherein:

figure 1 is a view of the pre-punched cardboard sheet for the execution of the container's outer box-like body, with the plastic bag indicated in dot-and-dash lines;

figure 2 is a view of the finished empty container in its flattened condition;

figure 3 is a view of the closure device in its completely open condition;

figure 4 is a view of the closure device during a step of its preparation for use;

figure 5 is a view of the upper portion of the container during its use;

figure 6 is a view of the container seen from its still-open bottom;

figure 7 is a view of a first step of the forming of the bottom;

figure 8 is a view of the finished container's bottom;

figure 9 is a view of the container's closure device according to another aspect of the invention.

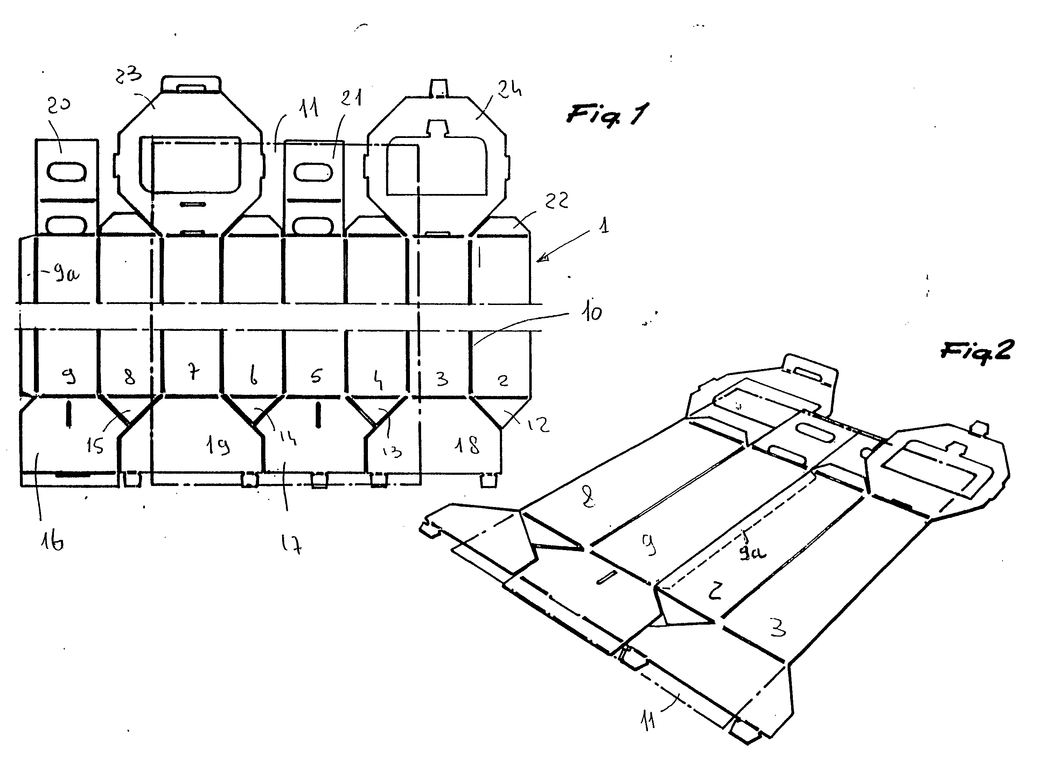

With reference to figures 1 to 8, the reference numeral 1 generally indicates the pre-punched cardboard sheet, preferably made of corrugated cardboard, which when appropriately folded is capable of constituting the outer box-like body of the container; said sheet comprises the surfaces 2, 3, 4, 5, 6, 7, 8 and 9, each whereof is intended to form one of the faces of the lateral wall; said surfaces are delimited by creases such as the one indicated by 10 provided between the surfaces 2 and 3.

The plastic bag 11 is associated with said surfaces by glueing; more precisely, said bag is placed on the cardboard sheet, on which a layer of glue has been applied beforehand, at the surfaces 4, 5, 6 and 7, so that said bag is rigidly coupled to the inside of said lateral wall when the closed lateral wall of the outer box-like body is formed by folding the cardboard sheet along the creases defined between the surfaces 7 and 8 and the surfaces 3 and 4 and by subsequently applying a pressure adapted to ensure the glueing of the peripheral band of the face 2 on the wing 9a and the glueing of the bag 11 on the cardboard.

The configuration assumed by the now completed container is the flattened one illustrated in figure 2, which characterizes it when it is empty and allows its easy storage.

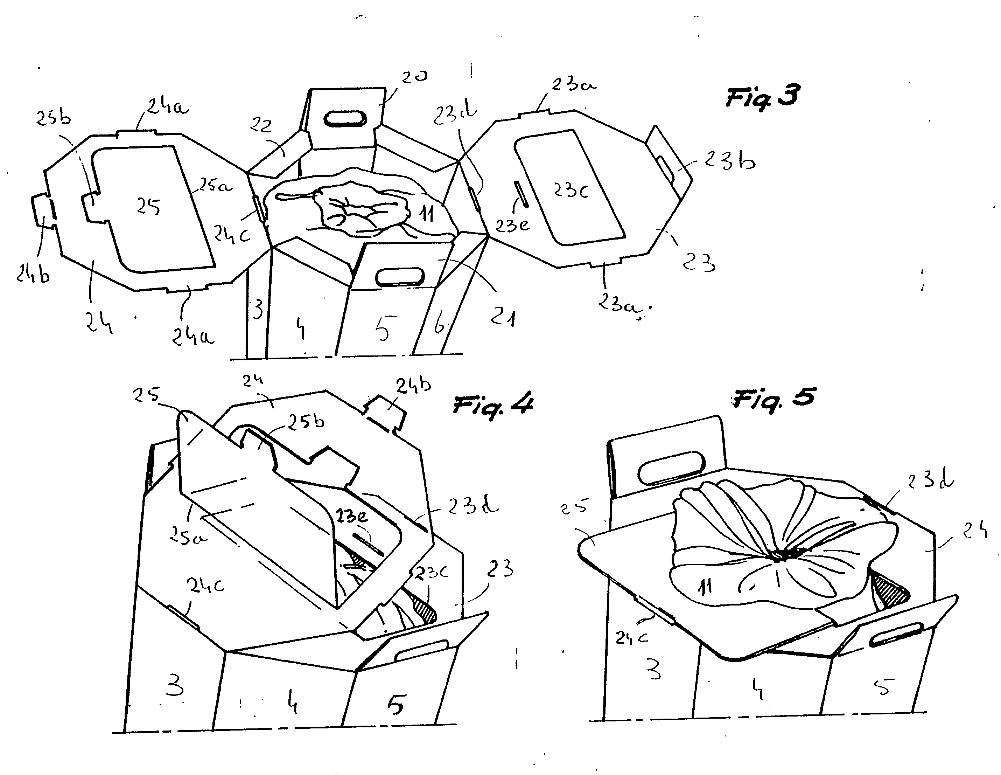

When the container is to be used, it is first of all opened out in the shape of a right prism with an octagonal base, with the lateral wall formed by eight faces constituted by the surfaces 2, 3, 4, 5, 6, 7, 8 and 9, as shown in figures 3 to 8; the bag 11 is also opened in this condition.

It is now necessary to form the bottom of the box-like body, and the four strips 12, 13, 14 and 15 are first provided for this purpose; said strips are monolithically articulated by means of creasing lines to the edge of a corresponding number of faces of the lateral wall and are folded immediately; a first pair of strips 16 and 17 furthermore extends from the opposite surfaces 9 and 5; said strips are folded and associated by inserting the tooth 17a in the slot 16a, as illustrated in figure 7. A second pair of strips 18 and 19 is furthermore provided; said strips extend respectively from the opposite surfaces 3 and 7 and are shaped so that when they are arranged adjacent they reproduce the shape of the bottom. Said strips are folded as indicated in figure 8 and are rigidly associated with the strips 16 and 17 by inserting the teeth 18a, 19a in the slot 16b provided in the strip 16 and by inserting the teeth 18b, 19b in the slot 17b provided in the strip 17.

The bottom thus formed is particularly compact and perfectly closed by virtue of the co-operation of all the described components.

It is then necessary to operate on the other base, where the handles 20 and 21 are formed first of all by folding the strips which are appropriately provided with slots which extend respectively from the surfaces 9 and 5 and by keeping them in position by subsequently folding the four strips 22 which are articulated to the edge of a corresponding number of faces and co-operate to strengthen the closure device which is now described with particular reference to figures 3, 4 and 5.

Said closure device comprises a first lid 23 which is monolithically articulated to the surface 7 by means of a creasing line and has wings 23a for supporting on the edges of other surfaces and an alignment tab 23b.

The opening 23c and the slots 23d, 23e are provided within the lid 23 for the purposes described hereafter.

The second lid 24 is monolithically articulated by means of a creasing line to the edge of the surface which is opposite to the articulation surface of the lid 23 and has wings 24a for supporting on the edges of other faces of the container and a tab 24b for locking it by insertion in the slot 23d; the reference numeral 24 furthermore indicates a slot which will be described hereafter during the description of the operation.

The flap 25 is provided within the lid 24 and is intended to superimpose on the opening 23c provided in the first lid, duplicating its shape and dimensions, when said two lids are superimposed on one another; said flap is monolithically articulated on the lid along the creasing line 25a and has the tab 25b which will be described hereafter.

The operation of the described closure device is very simple.

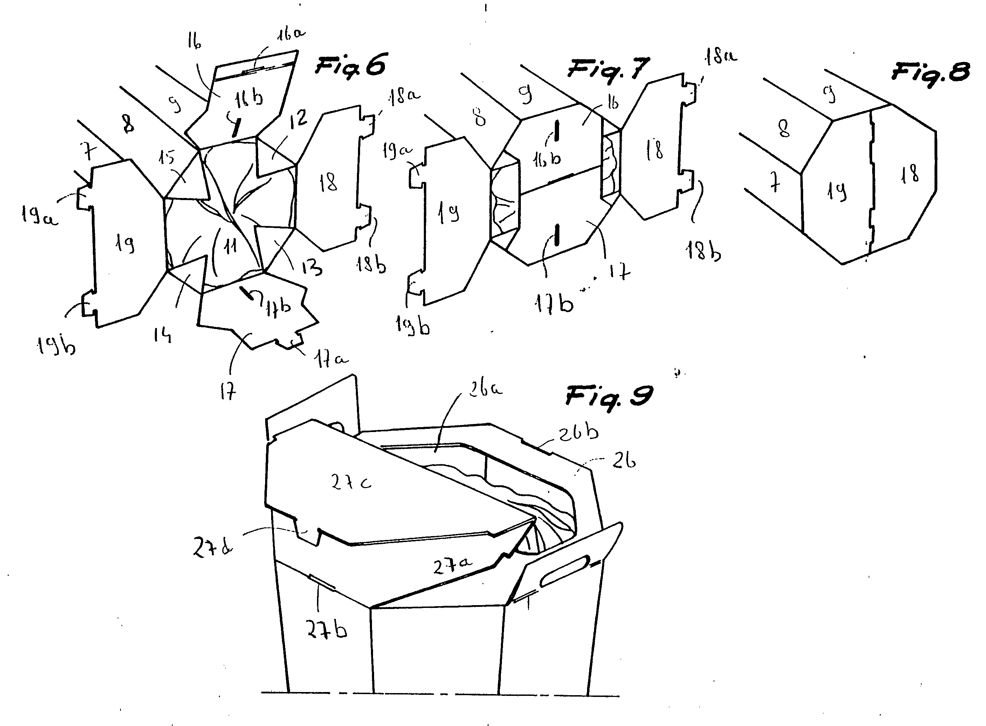

The first lid 23 is closed initially, and the second lid 24 is then closed and locked by inserting the tab 24b in the slot 23d; at this point the flap 25 is raised from its co-planar arrangement with the lid 24 and is rotated through 180° about the articulation line 25a until it makes contact with said lid and is locked by inserting the tab 25b in the slot 24c as illustrated in figure 5.

In this condition the opening 23c is open and the inlet of the bag 11 is caused to protrude therefrom, again as illustrated in figure 5; in this manner the container is ready to be filled.

Once the bag has been filled completely, the user ties the inlet of the bag by means of a lace, then presses on the inlet to push it into the box-like body, below the lids, and then rotates the flap 25 through 180°, moving it so as to close the opening 23c and locks it in this position by inserting the tab 25b in the slot 23e.

The container is perfectly closed and ready for removal.

Figure 9 illustrates a variation of the closure device according to the invention.

Said variation has a first lid 26 with an opening 26a and a slot 26b; said lid is articulated to a surface of the outer box-like body of the container at the side where the slot 26b is provided; said variation furthermore has a second lid which is provided with the portion 27a articulated to a surface of the box-like body at the side where the slot 27b is defined and the portion 27c articulated by means of a creasing line to the portion 27a; the reference numeral 27d indicates a tab which extends from the portion 27c.

The condition in which the opening 26a is not closed is obtained when the portion 27c adheres completely to the portion 27a, which is locked by the insertion of the tab 27d in the slot 27b, and it is possible to extract from said opening the inlet of the bag comprised within the container in order to fill it; when said bag is full the container is closed by rotating the portion 27c in the position which closes the opening 26a and is locked by inserting the tab 27d in the slot 26b.

The described container is therefore particularly suitable for collecting the waste which is progressively created in workplaces, and in particular the toxic waste of hospitals, such as pieces of gauze, syringes and the like, as mentioned, which can be inserted in the container without requiring the user to take particular care in the operation, by virtue of the large size of the bag's inlet allowed by the closure device according to the invention; once the container has been filled, the bag 11 is coupled to the outer cardboard box-like body so safely that its separation during transfers performed by gripping said box-like body by the handles can be ruled out, even if the box-like body's bottom deteriorates.

Another characteristic which makes the container according to the invention particularly efficient furthermore resides in the fact that the closure device is particularly simple and is therefore easy to handle for the operator both when forming it and when it is finally closed, and is suitable for any kind of container, regardless of the manner in which the remaining parts of the outer box-like body, in particular the lateral wall, are executed.

The described invention is susceptible to numerous modifications and variations, all of which are within the scope of the inventive concept; thus, for example, the opening provided in the first lid may have any shape, and the second lid may comprise, instead of a single flap 25 in the lid 24, a pair of flaps which are monolithically articulated to said lid by means of creased lines and can be locked in the position in which the opening provided in the first lid is open by means of tabs monolithically provided in said second lid.

The lateral wall of said outer box-like body may be formed by any number of faces, and in any case it may be configured in any manner; the bottom may likewise be configured in any manner.

In the practical embodiment of the invention, all the details may be replaced with other technically equivalent elements; the materials employed, as well as the shapes and dimensions, may furthermore be any.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Disposable container, comprising an outer box-like body made of cardboard or the like, with a lateral wall adapted to support a bag rigidly associated therewith, a bottom and a closure device at the face intended to be directed upwards, characterized in that said closure device comprises a first lid which is adapted to associate with the face of the container which is to be directed upwards and has an opening adapted to allow the bag's inlet to protrude, and furthermore comprises a second lid which is intended to superimpose on said first lid and has a portion adapted to be selectively locked in positions in which the opening provided in said first lid is closed or not closed.

2. Container according to claim 1, having the shape of a right prism with a polygonal base, characterized in that said first lid and said second lid completely cover the face of the container which is intended to be directed upwards, said lids being monolithically articulated by means of creased lines so as to extend from the edges of two opposite faces of the lateral wall, said lids having wings for supporting on the edges of other faces as well as alignment and fixing tabs.

3. Container according to claim 1, characterized in that the portion of the second lid which is adapted to be selectively locked in positions which close or do not close the opening provided in said first lid has the shape of a flap which is articulated by means of a creasing line and has a tab adapted to selectively insert in a slot provided in said second lid and in a slot provided in said first lid to respectively lock it in the condition in which said opening provided in said first lid is closed and not closed.

4. Container according to claim 1, characterized in that said second lid has a creasing line which defines an articulated portion adapted to be selectively locked in the position in which said opening provided in said first lid is closed or not closed by inserting a tab respectively in a slot provided in said first lid and in a slot provided in said second lid.

5. Container according to claim 1, characterized in that the portion of said second lid which is adapted to be selectively locked in a position in which said opening provided in said first lid is closed or not closed comprises a pair of flaps monolithically articulated to said lid on parallel creased lines, a tab being monolithically provided for each flap in said second lid and being adapted to lock said flap adherent to the lid in the position in which said opening is not closed by superimposing on said flap.

6. Container according to claim 1, characterized in that the outer box-like body comprises a lateral wall to which the bag is rigidly associated by glueing, said wall being obtained by folding a sheet of cardboard into the shape of a right prism with any number of faces, said cardboard sheet having creasings which define surfaces, each whereof is intended to form one of said faces, said box-like body being adapted to assume a flattened configuration when empty, a wing being furthermore provided which extends monolithically from one of said sides of said cardboard sheet which is parallel to the creasings, said wing being adapted to be rigidly associated by glueing with the opposite end of said sheet, so as to form the closed lateral wall of said box-like body.

7. Container according to one or more of the preceding claims, characterized in that the bottom of said outer box-like body comprises a first pair of strips, each whereof has at least one slot, said strips extending from sides of substantially opposite faces of the lateral wall and being adapted to mutually associate by virtue of the insertion of a tab extending from one of said strips into a slot provided in the other, said outer box-like body furthermore comprising a second pair of strips shaped so as to reproduce the shape of the bottom when they are arranged mutually adjacent, said strips also extending from sides of substantially opposite faces of the lateral wall, each of said strips having at least one tooth adapted to associate with the slots provided in the strips of the first pair.

8. Container according to one or more of the preceding claims, characterized by a pair of strips which extend from the sides of opposite faces of the lateral wall of the outer box-like body, said strips being monolithically articulated on creasing lines and being adapted to constitute the handles of the container.

9. Container according to one or more of the preceding claims, characterized in that some faces of the lateral wall of the outer box-like body have strips articulated on the side at the closure device.

10. Container according to one or more of the preceding claims, characterized in that some faces of the lateral wall of the outer box-like body have strips articulated on the side at the bottom.

Fig 1

Fig 2

EP 0 333 147 A1

Fig. 3

Fig. 4

Fig. 5

Fig.6

Fig.7

Fig.8

Fig.9

EP 0 333 147 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 534 489 (BARTLETT) <br> * Column 2, line 35 - column 3, line 11; column 3, line 32 - column 4, line 38; figures 1,4-6 * <br> --- | 1,2,6 | B 65 F 1/06 <br> B 65 D 5/10 <br> B 65 D 5/46 |
| A | GB-A-1 271 791 (HOOGENDIJK) <br> * Whole document * <br> --- | 1 | |
| A | DE-A-2 903 562 (HAMMERLIT) <br> --- | | |
| A | US-A-2 365 159 (WALTON) <br> --- | | |
| A | FR-A-1 215 335 (PASTOU) <br> --- | | |
| A | DE-U-8 608 885 (KAYSER) <br> ----- | | |

TECHNICAL FIELDS
SEARCHED (Int. Cl.4)

B 65 F
B 65 D

The present search report has been drawn up for all claims

| Place of search | Date of completion of the search | Examiner |
|---|---|---|
| THE HAGUE | 20-06-1989 | MARTENS L.G.R. |